# EUROPEAN PATENT SPECIFICATION

(11) **EP 2 037 941 B1**
(45) Date of publication and mention of the grant of the patent: **08.04.2015**
(21) Application number: 07795492.3
(22) Date of filing: 29.05.2007
(51) Int. Cl.: A61K 31/714, A61P 35/00, A61K 9/00

(54) **COBALAMIN COMPOSITIONS FOR TREATING OR PREVENTING MUCOSITIS**
COBALAMIN-ZUSAMMENSETZUNGEN ZUR BEHANDLUNG ODER PRÄVENTION VON MUCOSITIS
COMPOSITIONS DE COBALAMINE DESTINÉS AU TRAITEMENT OU À LA PRÉVENTION DE MUCOSITE

(30) Priority: 30.05.2006 US 809517 P; 31.07.2006 US 834641 P; 31.10.2006 US 855785 P
(43) Date of publication of application: 25.03.2009
(60) Divisional application: 15156860.7
(73) Proprietor: Orahealth Corporation, Bellevue, WA 98004 (US)
(72) Inventor: HALEY, Jeffrey, T., Bellevue, WA 98005-4211 (US)
(74) Representative: Ferreccio, Rinaldo
(86) International application number: PCT/US2007/012747
(87) International publication number: WO 2007/142973

(56) References cited:
- WO-A-02/064113
- WO-A-03/006072
- WO-A-03/103691
- WO-A1-03/002148
- US-A1- 2010 034 757
- DATABASE WPI Week 200154 Thomson Scientific, London, GB; AN 2001-489617 XP002528631 & CN 1 297 747 A (ZHU Z) 6 June 2001 (2001-06-06)
- MACPHAIL ET AL: "Topical and systemic therapy for recurrent aphthous stomatitis" SEMINARS IN CUTANEOUS MEDICINE AND SURGERY, W.B. SAUNDERS, PHILADELPHIA, US, vol. 16, no. 4, 1 December 1997 (1997-12-01), pages 301-307, XP005468195 ISSN: 1085-5629
- VOLKOV ILIA ET AL: "Case report: Recurrent aphthous stomatitis responds to vitamin B12 treatment." CANADIAN FAMILY PHYSICIAN MÉDECIN DE FAMILLE CANADIEN JUN 2005, vol. 51, June 2005 (2005-06), pages 844-845, XP002528630 ISSN: 0008-350X
- VOLKOV I. ET AL.: 'Vitamin B12 could be a "marker key" in the regulation of multiple pathological processes' JOURNAL OF NIPPON MEDICAL SCHOOL vol. 73, no. 2, 2006, pages 65 - 69, XP008092217
- BJOERKEGREN K. ET AL.: 'Reported symptoms and clinical findings in relation to serum cobalamin, folate, methylmalonic acid and total homocysteine among elderly Swedes: a population-based study' JOURNAL OF INTERNAL MEDICINE vol. 254, no. 4, 2003, pages 343 - 352, XP008091990
- DATABASE ESPACENET [Online] 'Antiulcer Medicine JP9208598'

## Description

### FIELD OF THE INVENTION

The present invention relates to compositions containing cobalamin suitable for topical delivery to moist epithelial surfaces for treatment of inflammatory, ulcerative and painful conditions such as mucositis, stomatitis, vestibulitis, aphthous ulcerations, and Behcet's syndrome.

### BACKGROUND OF THE INVENTION

Mucositis is an inflammation of the mucous membranes. Stomatitis is an inflammation of mucous membranes in the mouth. About 20 percent of people suffer from recurrent stomatitis in the form of aphthous ulcers, also called mouth ulcers, mouth sores, canker sores, denture sores, and sores from braces. Many women get oral aphthous ulceration at specific times of the menstrual cycle and some simultaneously get similar ulcers in the genital tract, in particular the vulva and vagina. This is sometimes very severe and can cause retention of urine and require strong painkillers and sedatives. The most severe form is called Behcet's syndrome.

Aggressive cancer treatment may have toxic effects on normal cells as well as cancer cells. The gastrointestinal tract, including the mouth, is especially affected because these cells are replaced by the body continuously. Mucositis in the mouth (stoamatitis), is one of the most common oral problems occurring after chemotherapy and radiation therapy. Oral mucositis can contribute to oral infections, inability to taste normally and pain arising from the resulting open sores that can develop. Oral mucositis can become so painful that the patient will not eat or drink, contributing to dehydration and malnutrition. Oral mucositis frequently also occurs in HIV patients, particularly when associated with Kaposi's sarcoma, in patients affected with non-Hodgkin's lymphoma, in debilitated elderly patients and in patients receiving BRM treatments like interleukin-2, TNF, interferons, lymphokine-activated lymphocytes and the like. Mucositis typically manifests as an erythematous, burn-like lesion or as random, focal-to-diffuse, ulcerative lesions. Stomatitis refers to an inflammatory reaction affecting the oral mucosa, with or without ulceration, that may be caused or intensified by pharmacological, particularly chemotherapeutic treatments, or by radiotherapy. Stomatitis can range from mild to severe; the patient with severe stomatitis is unable to take anything by mouth.

The Chinese patent application CN2001033378 describes a sucking medicine for treating stomatocace. The patent application WO-A1-03/006072 discloses antiulcer compositions and methods using a citrate and/or succinate salt as active agents. The publication Canadian Family Physician, 51, 2005, pages 844-845 describes a case report of treating stomatitis via parenteral injections of vitamin B12.

There is a need to provide method and compositions for treating and preventing inflammatory conditions of the moist epithelial surfaces, such as the mouth, particularly treatment of mouth sores, and other mucosa including vagina and rectum.

### SUMMARY OF THE INVENTION

The present invention fills this need by providing the use of troche compositions that topically apply cobalamin (vitamin B12) to epithelial tissues that are subject to mucositis. The cobalamin may be in the form of cyanocobalamin, which is not a bioactive form and must be converted to a bioactive form before it is used by human tissues. It may be a bioactive form of cobalamin, such as methylcobalamin (a/k/a mecobalamin), deoxyadenosylcobalamin (a/k/a adenosylcobalamin) or hydroxocobalamin.

The composition is a troche, such as a lozenge or "drop" as shown in Figure 1.. It is an adhering troche, such as a troche with adhesive on one side suitable for adhering to mucosa or to teeth or gums as shown in Figure 2. It may be an adhering troche that is thin and often flexible, called a patch, as shown in Figure 3.

The composition contains an amount of cobalamin that is effective for prevention or treatment when delivered topically to the mucosal tissues. For prevention, the delivery is typically at least once each day.

The troche comprisies bioactive cobalamin in a quantity effective to reduce mucositis when used regularly on moist epithelial tissues, i.e. at least 100 micrograms of bioactive cobalamin. The troche may be made adherent, perhaps using acacia gum as an adhesive, and it may be thin in the shape of a patch, perhaps comprising collagen which aids flexibility.

In another aspect, the invention uses an adhering troche comprising cyanocobalamin in a quantity effective to reduce oral mucositis when used regularly by dissolving in the mouth.

The invention is used for treating or preventing mucositis in a patient by regularly applying to a mucosal surface of a patient in need thereof an effective amount of a composition comprising cobalamin. The cobalamin may be selected from the group comprising cyanocoblalmin, methylcobalamin, hydroxocobalamin, and adenosylcobalamin. The composition may be a liquid, gel, paste, dissolving troche, suppository, or patch, including an oral patch. The composition comprises at least 100 micrograms of cobalamin. The composition may be administered at least once a day every day for prevention. The mucositis may occur in the mouth as stomatitis or aphthous ulcers. The mucositis may occur in the oro-pharynx or the oesophagus or the vagina or the rectum. The present invention can be more fully explained by reference to the following detailed description and illustrative examples.

### DRAWINGS

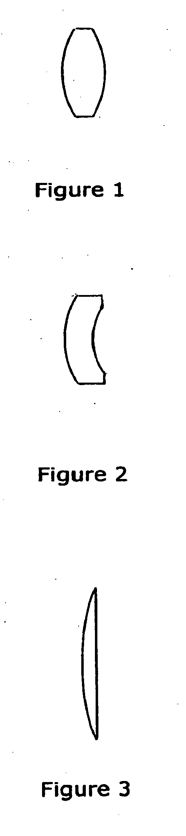

Figure 1 shows a troche in a typical shape, often called a lozenge. Figure 2 shows a cross section of an adhering troche in a shape designed to adhere to a tooth or orthodontic brace.
Figure 3 shows an edge view of a troche in the form of an oral patch designed to adhere to the cheek or lip.

### DETAILED DESCRIPTION OF THE INVENTION

Without being bound by a particular mode of action, the favorable therapeutic results obtained by the use of the compositions of the present invention are believed to be due to interactions between molecules of cobalamin with elements of the inflammatory process, which is best achieved with a topical application of bioactive cobalamin. It is helpful if the delivery vehicle can adhere to the oral mucosa providing a protective coating for the exposed nerve endings and holding the active molecules in topical application. Furthermore, the moisturizing or mucous releasing effects of some the compositions have beneficial effects as they protect mucous membranes from further irritations.

These compositions can be used by themselves or in an admixture with one or more medicaments, excipients and/or adjuvants, preferably forming a viscous and lubricating substance that remains adherent to the surface epithelium.

The troche compositions of the present invention are administered by topical application. In a particular embodiment the patient, after applying to the oral mucosa a dissolving troche, if desired, may refrain from eating or drinking for a certain time, ranging from minutes up to hours. Alternatively, the patient, if desired, may eat or drink immediately after applying the composition.

In the mouth, the troche may have a feel and texture like gummy candies. It may be made with slowly dissolving hydrocolloids so that that it typically lasts in the mouth for at least ten minutes to six hours. The troche can be formed in the shape of a tablet or a lozenge, as shown in Figure 1 , or a wafer or any other desired shape. A preferred shape is a thin lentil, nearly flat on one side, as shown in Figure 3, which may be called a patch. A detailed description of such a troche/patch and how to make it are disclosed by the same inventor in US patent application serial number 10/287,843 filed November 5, 2002.

To cause the troche to dissolve (erode) very slowly in saliva, a binder that dissolves slowly in saliva is incorporated. Binders that have been tested and found to work include carrageenan (preferably kappa), xanthan gum, xanthan gum combined with konjac gum, agar, and carboxymethylcellulose (CMC). Other gums similar to those listed, such as locust bean gum which has properties similar to konjac gum, and guar gum should also work, as well as starches, such as corn starch or, particularly pregelatinized corn starch, sometimes called zea mays.

A method of manufacturing troches in the shape of patches is to use gum drop manufacturing equipment, squirting a hydrated mixture heated above the gel melting temperature through nozzles onto a sheet of plastic or wax coated paper, allowing the troches to cool and gel, and drying the troches. The troches are preferably dried until the water activity level is lower than 0.8 so that the troches will not grow mold or other organisms. The drops are allowed to cool and then the sheets of plastic or coated paper with the drops on them are dried in a drying chamber. The product is sold still adhered to the plastic or paper and the user pulls it off the plastic or paper.

One troche formulation is made by combining cobalamin and xylitol with collagen and with other binder ingredients. Collagen, which is the organic molecule that makes up skin and the lining of the mouth (a form of skin), tends to adhere very well to itself, making it glutinous, and therefore adheres very well to the lining of the mouth. An effective and cost effective form of collagen is food grade gelatin which is made from animal skins.

Testing shows that, if the primary binders are xanthan gum and konjac gum, preferred dry weight formulations have between 10% and 50% xylitol, between 30% and 50% food grade gelatin, between 5% and 15% xanthan and konjac gums, between 5% and 30% cellulose fiber.

Other methods for making adherent troches include extrusion of a sheet and then die cutting. The cobalamin can be incorporated in other forms of troches including suppositories, lozenges, mints and other candies.

Troches in the form of dissolving muco-adhesive tablets may be made by pressing powders including mucoadhesive hydrocolloids. A preferred form of such an adhering troche may be made by mixing dry powders consisting of 80 percent acacia gum (gum arabic) for adhesive and 20 percent xylitol for flavor, then adding up to 1 percent carboxymethylcellulose (CMC), to enhance mouth feel, moderate adhesiveness of the acacia gum, and slow dissolution, and about 500 micrograms of cobalamin. The tablet may be pressed into the shape shown in Figure 3 for good adhesion to a tooth. Such a tablet is preferably about 8 mm in diameter and about 85 milligrams in weight.

The preferred instruction to users is to stick a troche in their mouths, just before they go to sleep, between teeth and cheek, placing it on the tongue side of the teeth if the patient suffers particularly from ulcers on or under the tongue and otherwise placing it on the cheek side. In greater detail, the instructions state: "Use after brushing teeth, at least one disc per day, and allow to dissolve for 30 minutes with nothing else in your mouth. In an area of your mouth where you experience sores, use your tongue to hold the dimpled side of the disc against a tooth until it adheres. The disc will slowly dissolve over 10 to 40 minutes, releasing cobalamin which is absorbed directly into the mouth lining tissues. When you first start using the discs, use three discs the first day to quickly boost the level of cobalamin in your mouth tissues. Thereafter, use at least one disc each day. If you miss a day, use at least two discs the next day. If you miss two or more days, begin with three discs the first day you resume. Vary the spots you place the disc from day to day in the places where you frequently experience sores, such as a front tooth against your lip, on the inside of a lower tooth to protect under your tongue, and on the outside of a molar in each cheek. If you have a sore, place the disc near the sore."

Such discs were given to test subjects with the above instructions, generating the following results: Fifteen subjects with minor RAU at least monthly were given 30 adhering dissolving discs and instructed to use one each day. Seven received discs with 500 meg methylcobalamin and eight received discs with red food coloring. In the placebo group, two out of eight (25%) reported a benefit consisting of reduced frequency of minor RAU, reduced duration of ulcers, or reduced peak pain levels; two out of eight were unsure; and four out of eight (50%) were confident they experienced no benefit. In the active group, all seven (100%) reported a benefit. Twenty-five additional non-blinded subjects were given active discs with 500 meg methylcobolamin. Approximately 3 to 7 days of daily use was required for the preventive effects to reach full strength. Twenty- four subjects (96%) reported a benefit of using the discs. Five subjects who reported a benefit stopped using the discs and all five reported that, within 4 to 7 days (mean=4.8), they each had an ulcer with a pain level like before they started using the discs.

Other tests were run with cyanocobalamin in oral patches and in a gel with similar results. The gel formulation was about 93.5% water, 0.5% Ticalose carboxymethylcellulose, about 6% xylitol for flavor, and about 0.05% cyanocobalamin.

The composition is intended to release an effective amount of cobalamin molecules in the mouth to treat or prevent stomatitis, particularly including aphthous ulcers. The compositions can comprise a pharmaceutically acceptable excipient, preferably for topical administration, such as one or more of the following: viscosity-increasing agent; surfactant; stabilizing agent/preservative; flavor, fragrance, sweetening agent; bioadhesive; co-solubilizer.

Examples of said excipients comprise cellulose derivatives, acrylic or methacrylic acids polymers or copolymers, ethylene or propylene glycols, polyethoxylated hydrogenated castor oil, EDTA, sodium benzoate, sodium or potassium sorbate, dextrins, sodium saccharin, aspartame and other excipients conventionally used in the formulation of coilutories or liquid oral forms. Oral formulations can include standard carriers such as pharmaceutical grades of mannitol, sorbitol, xylitol, lactose, starch, magnesium stearate, sodium saccharine, cellulose, magnesium carbonate, etc. Additional examples of suitable excipients are described in "Remington's Pharmaceutical Sciences" by E. W. Martin.

The compositions may further comprise one or more other active ingredients, such as an antibacterial, disinfectant, antifungal, analgesic, other anti-inflammatory, emollients, local anesthetics and the like. Suitable antimicrobials include, but are not limited to, quaternary ammonium salts such as benzalkonium chloride. The treatment or prevention of mucositis inflammation in a patient in accordance with the invention comprises administering to a patient in need thereof an effective amount of a composition comprising at least 100 micrograms of cobalamin in troche from. The composition may be administered at least once daily for at least three consecutive days. In yet another embodiment, the composition is administered at least once daily for at least seven consecutive days. In yet another embodiment, the composition is administered at least once daily every day without end to prevent or reduce the effects of mucositis. In addition to its ordinary meaning, the term treatment encompasses inhibition of progression of symptoms or amelioration of symptoms of inflammation and mucositis. TThese methods may provide an effective therapeutic or preventive treatment for mucositis and stomatitis of various origin and severity and, more generally, of the lesions of the oro-pharynx cavity and oesophagus, particularly those caused by recurrent aphthous ulceration, dental devices, by radio- or chemotherapy, and by surgery. The precise dose to be employed in the composition will depend on the route of administration, and the seriousness of the disease or disorder, and should be decided according to the judgment of the practitioner and each patient's circumstances. The treatment can be protracted until remission of symptoms, usually for at least 2 days, but preferably 5-10. More prolonged treatments are not contraindicated, considering zero toxicity of the components of the formulations, and once daily use as a preventive is effective for many conditions.

The composition may be provided a pharmaceutical pack or kit comprising one or more containers. In an embodiment, the compositions can be presented as single- or multi-dose forms in a flexible packet

### EXAMPLE 1

Qualitative-quantitative composition percent composition, delivered in a bottle or packet: about 1.5 grams of dry powder comprising, by dry weight, about 40% Ticalose cellulose, about 58% xylitol for flavor, and about 2% methylcobalamin or hydroxocobalamin. The consumer then adds about 50 grams of water and lets the mixture sit until the cellulose is fully hydrated to make a gel.

### EXAMPLE 2

Qualitative-quantitative composition percent composition: Ingredient % By Weight: bioactive cobalamin (methylcobalmin) 0.2, partially evaporated aloe vera sap with preservative: the balance. For a concentrated version of the invention, 10 ml to 30 ml of the above composition is distributed in a packet or bottle.

### EXAMPLE 3

Qualitative-quantitative composition percent composition: Ingredient % By Weight: bioactive cobalamin (methylcobalmin) 0.2, glycerin 40, guar gum 9.0, water: the balance. For a concentrated version of the invention, 10 ml or 15 ml of the above composition is distributed in a packet or mono-dose vial.

### EXAMPLE 4

Qualitative-quantitative composition percent composition: Ingredient % By Weight: bioactive cobalamin (methylcobalmin) 0.2, PVP (K60 to K100) 8.0, Maltodextrin 4.0, Propylene glycol 2.9 Potassium sorbate 0.3, Sodium benzoate 0.3, Hydroxyethyl cellulose 1.5, Hydrogenated castor oil PEG-40 0.3, Disodium EDTA 0.1, Benzalkonium chloride 0.5, Perfume 0.16, Xylitol 1 , Depurated water: the balance.

EXAMPLE 5 Manufacturing of an oral patch containing methylcobalamin The following were mixed together in the percentages listed by dry-weight: Methylcobalamin 1.35% Konjac and Xanthan gum 9.0% Xylitol - 22% Cellulose 26%

### Gelatin 41.6%

To this dry mixture 5 times by weight of water was added and the mixture stirred and heated to 140 - 200° F so as to produce a uniform viscous liquid. Drops of the mixture were deposited onto sheets to form blobs at a temperature of about 140° F. The resultant oral patches each containing 500 micrograms methylcobalamin were then dried at room temperature.

## Claims

1. An adherent troche composition for use in a method of treating mucositis by topical application on moist epithelial tissues, comprising at least 100 micrograms of cobalamin, said quantity being effective to reduce or prevent mucositis when applied on affected tissues.

2. A composition for the use according to claim 1, wherein the cobalamin is selected from methylcobalamin, hydroxycobalamin, adenosylcobalamin and cyanocobalamin.

3. A composition for the use according to claims 1-2, comprising acacia gum as an adhesive.

## Patentansprüche

1. Anhaftende Pastillenzusammensetzung zur Verwendung in einem Verfahren zur Behandlung einer Mucositis durch topische Anwendung auf feuchten Epithelgeweben, mindestens 100 Mikrogramm Cobalamin enthaltend, wobei diese Menge dahingehend wirksam ist, bei Auftragung auf befallene Gewebe die Mucositis zu reduzieren oder zu verhindern.

2. Zusammensetzung für die Verwendung nach Anspruch 1, wobei das Cobalamin ausgewählt ist aus Methylcobalamin, Hydroxycobalamin, Adenosylcobalamin und Cyanocobalamin.

3. Zusammensetzung für die Verwendung nach den Ansprüchen 1 bis 2, umfassend Gummi arabicum als Haftmittel.

## Revendications

1. Composition de pastille adhésive destinée à être utilisée dans un procédé de traitement d'une mucosite par une application topique sur des tissus épithéliaux humides, comprenant au moins 100 microgrammes de cobalamine, ladite quantité étant efficace pour réduire ou empêcher une mucosite une fois qu'elle a été appliquée sur des tissus touchés.

2. Composition destinée à être utilisée selon la revendication 1, dans laquelle la cobalamine est sélectionnée dans le groupe constitué par la méthylcobalamine, l'hydroxycobalamine, l'adénosylcobalamine et la cyanocobalamine.

3. Composition destinée à être utilisée selon les revendications 1 ou 2, comprenant de la gomme arabique en tant qu'adhésif.
